Europäisches Patentamt

European Patent Office

Office européen des brevets

(11) Publication number: **0 382 214 B1**

# EUROPEAN PATENT SPECIFICATION

(45) Date of publication of patent specification: **03.05.95** (51) Int. Cl.⁶: **C12N 5/00**

(21) Application number: **90102491.9**

(22) Date of filing: **08.02.90**

(54) **Method of cell culture.**

(30) Priority: **10.02.89 JP 31844/89**

(43) Date of publication of application:
**16.08.90 Bulletin 90/33**

(45) Publication of the grant of the patent:
**03.05.95 Bulletin 95/18**

(84) Designated Contracting States:
**CH DE FR GB LI NL SE**

(56) References cited:
**WO-A-88/08448**

**PATENT ABSTRACTS OF JAPAN, vol. 8, no. 205 (C-243)[1642], 19th September 1984;& JP-A-59 95 930 (KOGYO GIJUTSUIN (JAPAN) 02-06-1984**

**CHEMICAL ABSTRACTS, vol. 107, no. 17, 26th October 1987, page 339, abstract no.150296t, Columbus, Ohio, US; & JP-A-62 06 682 (CANON K.K.) 13-01-1987**

(73) Proprietor: **KAO CORPORATION**
**14-10, Nihonbashi Kayaba-cho 1-chome**
**Chuo-ku**
**Tokyo (JP)**

Proprietor: **TOKYO WOMEN'S MEDICAL COLLEGE**
**8-1, Kawata-cho,**
**Shinjuku-ku**
**Tokyo 162 (JP)**

(72) Inventor: **Okano, Teruo**
**6-12-12, Konodai**
**Ichikawa-shi,**
**Chiba (JP)**
Inventor: **Kataoka, Kazunori**
**Kashiwa Birejji 141-9,**
**1083-4, Omuro**
**Kashiwa-shi,**
**Chiba (JP)**
Inventor: **Yamada, Noriko**
**Maenocho Haitsu 1-601,**
**6-10 Maeno-cho**
**Itabashi-ku,**
**Tokyo (JP)**
Inventor: **Sakurai, Yasuhisa**
**3-17-6 Eifuku,**
**Suginami-ku**
**Tokyo (JP)**

Inventor: **Amiya, Takayuki**
**Siti Haitsu Arumu 201,**
**2-11-3, Funatsu-cho**
**Wakayama-shi,**
**Wakayama (JP)**
Inventor: **Mamada, Akira**
**Asahi Puraza Nakanoshima 604,**
**289-1, Nakanoshima**
**Wakayama-shi,**
**Wakayama (JP)**


74 Representative: **VOSSIUS & PARTNER**
**Postfach 86 07 67**
**D-81634 München (DE)**

## Description

The present invention relates to a method for culturing cells, e.g. in the field of biology, medical science or immunology.

Hitherto, culturing cells has been conducted on a surface of glass or a surface-treated synthetic polymer. For example, a polystyrene which has been subjected to a surface treatment, e.g. $\gamma$ ray irradiation or silicon coating, is used as a bed material for cell culture. Cultured cells on the bed are collected or detached from the surface of the bed by treating with a proteolysis enzyme (e.g. trypsin) or a chemical material (e.g. EDTA). In the treatment with the proteolysis enzyme or chemical material, however, following problems are associated: (1) the treating process is complicated and there is a high possibility in introducing impurities. (2) The cultured or grown cells are adversely affected by the treatment and their inherent functions may be hurt.

In order to overcome the above-mentioned problems, the present invention uses a bed material from which cultured or grown cells are collected or detached without a proteolysis enzyme or chemical material. The bed material used according to the present invention comprises a support and a coating thereon, wherein the coating is formed from a polymer or copolymer which has a critical solution temperature to water within the range of 80 to 0 °C.

The critical solution temperature is defined as follows. When a certain material is mixed with water, the mixture is divided into two layers at a particular temperature because of poor solubility, but eventually the material is completely solved with water to turn to a uniform solution if it is either heated or cooled to pass a certain temperature. The certain temperature is defined as "critical solution temperature". If the uniform solution is formed when heated, the critical solution temperature is called "upper critical solution temperature". If the uniform solution is formed when cooled, it is called "lower critical solution temperature".

It is general that the critical solution temperature is determined by making a solution phase diagram to water (ion exchanged water or distilled water). For making the solution phase diagram, mixtures of a polymer to be measured and water in various concentrations (such as weight %, volume %, molar %, molar ratio etc.) are prepared and the mixtures are heated or cooled to observe the conditions of the mixture. The conditions are determined by art-known methods, such as (a) observing by eyes, (b) observing critical opalescence, (c) observing scattered light strength, or (d) observing transmitted laser light.

The polymer or copolymer used according to the present invention should have either an upper or lower critical solution temperature within the range of 80 to 0 °C, preferably 50 to 20 °C. If it is higher than 80 °C, the cultured or grown cells may die. If it is lower than 0 °C, growth rate of cells may be very low or the cells may die.

The polymer or copolymer used according to the present invention may be prepared by polymerizing or copolymerizing some hydrophilic monomers. Non-limited examples of the monomers, provided that parentheses indicate a lower critical solution temperature of homopolymer, are a (meth)acrylamide, such as acrylamide and methacrylamide; an N-substituted (meth)acrylamide, such as N-ethyl acrylamide (72 °C), N-n-propyl acrylamide (21 °C), N-n-propyl methacrylamide (27 °C), N-isopropyl acrylamide (32 °C), N-isopropyl methacrylamide (43 °C), N-cyclopropyl acrylamide (45 °C), N-cyclopropyl methacrylamide (60 °C), N-ethoxyethyl acrylamide (about 35 °C), N-ethoxyethyl methacrylamide (about 45 °C), N-tetrahydrofurfuryl acrylamide (about 28 °C) and N-tetrahydrofurfuryl methacrylamide (about 35 °C) ; an N,N-di-substituted (meth)acrylamide, such as N,N-dimethyl (meth)acrylamide, N,N-ethylmethyl acrylamide (56 °C), N,N-diethyl acrylamide (32 °C), 1-(1-oxo-2-propenyl)-pyrrolidine (56 °C), 1-(1-oxo-2-propenyl)-piperidine (about 6 °C), 4-(1-oxo-2-propenyl)-morpholine, 1-(1-oxo-2-methyl-2-propenyl)-pyrrolidine, 1-(1-oxo-2-methyl-2-propenyl)-piperidine and 4-(1-oxo-2-methyl-2-propenyl)-morpholine; and a vinyl ether, such as methyl vinyl ether (35 °C). A copolymer of the above-listed monomers and other monomers, a graft polymer or copolymer or a mixture of the polymers can also be employed in the present invention, in order to adjust a critical solution temperature depending upon the sort of cells, to enhance an interaction between the support and the coating thereon or to control the balance between hydrophilic and hydrophobic properties of the bed material. The polymer or copolymer used according to the present invention may be crosslinked unless inherent properties of the polymer are hurt.

The support of the present invention can be prepared from any material, for example polymers (e.g. polystyrene and poly(methyl methacrylate)), ceramics, metal, glass, modified glass. The shape of the support is not limited, but typically Petri dish, plate, fiber, particle, any containers for cell culture (e.g. flask).

A polymer or copolymer can be bound on the support in a chemical method or a physical method. In the chemical method, electron beam, $\gamma$ ray irradiation, ultraviolet irradiation, corona treatment and plasma treatment can be used. In a case where the support and the coating have groups reactive with each other, organic reaction (e.g. radical, anionic or cationic reaction) can also be used. In the physical method, the polymer per se or a combination of the polymer and a matrix compatible with the support is coated on the support, thus binding by physical absorption power. Examples of the matrix are a graft or a block copolymer of the polymer to be coated, with the monomer forming the support or other monomers compatible with the support.

In order to collect or detach the grown or cultured cells, the bed material is either heated or cooled to pass the upper or lower critical solution temperature, thus detaching the cells, and rinsed with an isotonic solution to collect.

The function of the present invention will be illustrated by adopting poly(N-isopropyl acrylamide) as an example. It is known that poly(N-isopropyl acrylamide) has a lower critical solution temperature of about 32 °C in water. The monomer, i.e. N-isopropyl acrylamide, is polymerized on a polystyrene Petri dish for cell culture by irradiating electron beam. At a higher temparature than 32 °C, the poly(N-isopropyl acrylamide) coating is hydrophobic and expels water molecules inside the coating to result in reducing its volume. At a lower temperature than 32 °C, the coating is hydrophilic and holds water molecules to result in swelling.

According to the present invention, the surface of the bed material reversibly changes hydrophilic to hydrophobic, vice versa, by controlling temperature. Accordingly, the grown or cultured cells are detached from the bed material by simply controlling temperature without destroying the cells, and then rinsed with an isotonic solution to collect. Since the bed material used according to the present invention does not employ the proteolysis enzyme (trypsin) and the chemical material (EDTA), the detaching or removing process is simplified and introduction of impurities is entirely excluded. The bed material used according to the present invention does not injure the cells and protects the inherent functions of the cells.

EXAMPLES

The present invention is illustrated by the following examples which, however, are not to be construed as limiting the invention to their details.

Examples 1 to 3

A Petri dish (available from Becton Dickinson Labware Co., Ltd. as FALCON 3001) was employed as a support and the cells to be cultured are bovine aorta endothelial cells. N-isopropyl acrylamide was dissolved in isopropyl alcohol in the concentration shown in Table 1 and coated on the Petri dish. It was then irradiated with electron beam in an irradiation dose shown in Table 1 to form a poly(N-isopropyl acrylamide) coating. The coated Petri dish was rinsed with ion-exchanged water to remove remaining monomers and dried in a clean bench.

Culturing bovine aorta endothelial cells was carried out at 37 °C in 5 % carbon dioxide using a Dulbecco's modified Eagle's medium (DMEM) containing 20 % fetal calf serum (FCS) on the obtained Petri dish. After growing cells, the Petri dish was cooled to 4 °C and allowed to stand to detach the cells. A collection % of the grown cells (number of cells collected by detaching / number of cells to be grown X 100) was calculated and the results are also shown in Table 1.

Examples 4 and 5

A coated Petri dish was prepared as generally described in Examples 1 to 3, with the exception that N,N-diethyl acrylamide was employed in the concentration shown in Table 1 instead of N-isopropyl acrylamide, and the experiment of Examples 1 to 3 was repeated. A collection % of grown cells is shown in Table 1.

Comparative Examples 1 to 3

In Comparative Example 1, no surface coating was conducted on a same Petri dish and an experiment was done as generally described in Example 1.

In Comparative Examples 2 and 3, an electron beam was irradiated on a same Petri dish which was not coated with any monomer solution, and an experiment was done as generally described in Example 1. A collection % of grown cells is shown in Table 1.

Table 1

| | Concentration of monomer or polymer | Irradiation dose of electron beam (Mrad) | Collection % of grown cells |
|---|---|---|---|
| Example 1 | N-isopropyl acrylamide 1 wt% | 5 | >90 % |
| 2 | Poly(N-isopropyl acrylamide) 1 wt% | 5 | >90 % |
| 3 | N-isopropyl acrylamide 10 wt% | 25 | >90 % |
| 4 | N,N-diethyl acrlyamide 1 wt% | 5 | >90 % |
| 5 | N,N-diethyl acrylamide 10 wt% | 5 | >90 % |
| Comp. Ex.1 | 0 | 0 | Detachment |
| Comp. Ex.2 | 0 | 5 | and collection |
| Comp. Ex.3 | 0 | 25 | are impossible |

In the above Examples and Comparative Examples, a contact angle was determined by an under water method using a FACE Contact Angle Meter CA-D (available from Kyowa Surface Chemistry Co., Ltd.) and the results are shown in Table 2.

Table 2

| | Contact angle at 37 °C | Contact angle at 4 °C |
|---|---|---|
| Example 1 | 42 | 25 |
| 2 | 48 | 28 |
| 3 | 36 | 22 |
| 4 | 44 | 24 |
| 5 | 46 | 26 |
| Comparative Example 1 | 58 | 64 |
| 2 | 56 | 62 |
| 3 | 56 | 62 |

Example 6

A coated Petri dish was prepared as generally described in Examples 1 to 3, with the exception that N-isopropyl methacrylamide was employed in the concentration shown in Table 3 instead of N-isopropyl acrylamide. Bovine aorta endothelial cells were cultured at 45 °C and cooled to 30 °C at which the cells were detached from the dish. A collection % of grown cells is shown in Table 3.

Example 7

A coated Petri dish was prepared as generally described in Examples 1 to 3, with the exception that N-n-propyl acrylamide was employed in the concentration shown in Table 3 instead of N-isopropyl acrylamide. Bovine aorta endothelial cells were cultured at 25 °C and cooled to 10 °C at which the cells were detached from the dish. A collection % of grown cells is shown in Table 3.

Table 3

| | Monomer concentration | Irradiation dose of electron beam (Mrad) | Collection % of grown cells |
|---|---|---|---|
| Example 6 | N-n-propyl acrylamide 10 % by weight | 25 | >90 % |
| Example 7 | N-isopropyl acrylamide 10 % by weight | 25 | >90 % |

Example 8

This example confirms an injured degree of the detached cells.

The detached cells of Example 3 were centrifuged at 600 G for 5 minutes to collect $1 \times 10^5$ cells which were then cultured on a FALCON 3001 Petri dish. Culturing process was the same as Example 3. After 4 days, number of cells were counted and the result is shown in Table 4.

Comparative Example 4

The cells cultured or grown in Comparative Example 1 were treated with a 0.05 % trypsin - 0.02 % EDTA solution to detach them. The detached cells were centrifuged at 600 G for 5 minutes to collect $1 \times 10^5$ cells which were then cultured on a FALCON 3001 Petri dish as generally described in Example 1. After four days, number of cell were counted and the result is shown in Table 4.

Table 4

| | Number of cells when culturing started | Number of cells after 4 days |
|---|---|---|
| Example 8 | $1\times10^5$ | $1\times10^6$ |
| Comp. Ex. 4 | $1\times10^5$ | $5\times10^5$ |

As is apparent from the above Examples and Comparative Examples, in the polystyrene Petri dish which was coated with poly(N-isopropyl acrylamide) or poly(N,N-diethyl acrylamide), a contact angle decreased by changing from 37 °C to 4 °C, as shown in Table 2. This shows that the coating on the dish changes from hydrophobic to hydrophilic. In Examples 1 to 5 in which the coated Petri dishes were employed, the cultured cells were easily detached from dishes when cooled, as shown in Table 1.

On the other hand, in case where such a coating is not coated, a contact angle is not changed when cooled, as shown in Table 2, thus the surface maintaining hydrophobic. If the dish which was not coated was employed, the detachment of the cultured cells was not observed.

As to the injured degree of the cultured cells, Example 8 could grow 10 times more than the stating point, but Comparative Example 4 could grow only 5 times more. This shows that an injured degree is smaller than a conventional one.

**Claims**

1. A method for culturing cells which comprises coating a support with a polymer or copolymer which has a lower or upper critical solution temperature to water within the range of 0 to 80 °C, culturing cells at a higher temperature than said lower critical solution temperature or at a lower temperature than said

EP 0 382 214 B1

upper critical solution temperature on said coated surface having hydrophobic properties and detaching and collecting the cultured cells from said coated surface by changing the temperature to a lower temperature than said lower critical solution temperature or at a higher temperature than said upper critical solution temperature, thereby changing the properties of said coated surface from hydrophobic to hydrophilic.

2. The method for culturing cells according to claim 1 wherein said polymer or coplymer has a lower or upper critical solution temperature to water within the range of 20 to 50°C.

3. The method for culturing cells according to claim 1 or 2 wherein said polymer or copolymer is selected from the group consisting of poly(N-isopropyl acrylamide), poly(N-isopropyl methacrylamide), poly(N-n-propyl acrylamide) and poly(N,N-diethyl acrylamide).

4. The method for culturing cells according to any one of claims 1 to 3 wherein said support is prepared from polystyrene or glass.

5. The method for culturing cells according to any one of claims 1 to 4 wherein said support is a Petri dish.

**Patentansprüche**

1. Verfahren zur Züchtung von Zellen, umfassend die Beschichtung eines Trägers mit einem Polymer oder Copolymer, das eine untere oder obere kritische Lösungstemperatur gegenüber Wasser innerhalb des Bereichs von 0 bis 80°C aufweist, Züchten von Zellen bei einer höheren Temperatur als die untere kritische Lösungstemperatur oder einer niedrigeren Temperatur als die obere kritische Lösungstemperatur auf der beschichteten Oberfläche mit hydrophoben Eigenschaften und Ablösen und Sammeln der gezüchteten Zellen von der beschichteten Oberfläche durch Ändern der Temperatur auf eine geringere Temperatur als die untere kritische Lösungstemperatur oder eine höhere Temperatur als die obere kritische Lösungstemperatur und dabei Verändern der Eigenschaften der beschichteten Oberfläche von hydrophob auf hydrophil.

2. Verfahren zur Züchtung von Zellen nach Anspruch 1, wobei das Polymer oder Copolymer eine untere oder obere kritische Lösungstemperatur gegenüber Wasser im Bereich von 20 bis 50°C aufweist.

3. Verfahren zur Züchtung von Zellen nach Anspruch 1 oder 2, wobei das Polymer oder Copolymer ausgewählt ist aus Poly(N-isopropylacrylamid), Poly-(N-isopropylmethacrylamid), Poly(N-n-propylacrylamid) und Poly(N,N-diethylacrylamid).

4. Verfahren zur Züchtung von Zellen nach einem der Ansprüche 1 bis 3, wobei der Träger aus Polystyrol oder Glas hergestellt wird.

5. Verfahren zur Züchtung von Zellen nach einem der Ansprüche 1 bis 4, wobei der Träger eine Petrischale ist.

**Revendications**

1. Procédé de culture de cellules qui comprend le revêtement d'un support par un polymère ou copolymère qui possède une température critique de solution inférieure ou supérieure par rapport à l'eau dans la gamme allant de 0 à 80°C, la culture des cellules à une température plus élevée que ladite température critique inférieure de solution ou à une température plus faible que ladite température critique supérieure de solution sur ladite surface revêtue présentant des propriétés hydrophobes, ainsi que le détachement et le recueil des cellules cultivées à partir de ladite surface revêtue par uné modification de la température à une température plus faible que ladite température critique inférieure de solution ou à une température plus élevée que ladite température critique supérieure de solution, faisant ainsi passer les propriétés de ladite surface revêtue de l'hydrophobie à l'hydrophilie.

2. Procédé de culture de cellules selon la revendication 1, dans lequel ledit polymère ou copolymère possède une température critique de solution inférieure ou supérieure par rapport à l'eau dans la

7

gamme allant de 20 à 50 °C.

3. Procédé de culture de cellules selon la revendication 1 ou 2, dans lequel on choisit ledit polymère ou copolymère dans le groupe constitué du poly(N-isopropylacrylamide), du poly(N-isopropylméthacrylamide), du poly(N-n-propylacrylamide) et du poly(N,N-diéthylacrylamide).

4. Procédé de culture de cellules selon l'une quelconque des revendications 1 à 3, dans lequel on prépare ledit support à partir de polystyrène ou de verre.

5. Procédé de culture de cellules selon l'une quelconque des revendications 1 à 4, dans lequel ledit support est une boîte de Petri.